# EUROPÄISCHE PATENTSCHRIFT

(11) **EP 3 830 062 B1**
(45) Veröffentlichungstag und Bekanntmachung des Hinweises auf die Patenterteilung: **18.02.2026**
(21) Anmeldenummer: 19745187.5
(22) Anmeldetag: 02.08.2019
(51) Int. Cl.: C07C 7/04, B01D 3/00, C07C 5/25, C07C 7/148, C07C 11/08, C07C 11/09, C07C 9/12, B01D 3/14

(54) **VERFAHREN ZUR AUFREINIGUNG VON ISOBUTEN AUS EINEM C4-STROM UND VERFAHRENSTECHNISCHE ANLAGE DAFÜR**
METHOD FOR THE PURIFICATION OF ISOBUTYLENE FROM A C4 FLOW AND PROCESS TECHNOLOGY FOR SAME
PROCÉDÉ DE PURIFICATION D'ISOBUTÈNE D'UN FLUX C4 ET INSTALLATION TECHNIQUE CORRESPONDANTE

(30) Priorität: 02.08.2018 EP 18187133
(43) Veröffentlichungstag der Anmeldung: 09.06.2021
(73) Patentinhaber: OMV Downstream GmbH, 1020 Wien (AT); BASF SE, 67056 Ludwigshafen am Rhein (DE)
(72) Erfinder: THOTLA, Suman, 68165 Mannheim (DE); CZAJKA, Pawel Tadeusz, 68161 Mannheim (DE); ISELBORN, Stefan Manfred, 67227 Frankenthal (DE); WEMHÖNER, Kai-Uwe, 1020 Wien (AT); POPP, Jürgen, 1020 Wien (AT)
(74) Vertreter: SONN Patentanwälte GmbH & Co KG
(86) Internationale Anmeldenummer: PCT/EP2019/070859
(87) Internationale Veröffentlichungsnummer: WO 2020/025782

(56) Entgegenhaltungen:
- EP-B1- 1 200 378
- DE-A1- 19 801 089
- US-A- 5 536 887
- US-B1- 6 215 036

## Beschreibung

Die Erfindung betrifft ein Verfahren und eine verfahrenstechnische Anlage zur Aufreinigung von Isobuten aus einem C4-Strom, der zumindest 1-Buten, 2-Buten, Isobutan und Isobuten aufweist.

Die Anforderungen an die Reinheit von Isobuten als Ausgangsstoff für verschiedene Produkte wachsen ständig. Besondere Reinheit wird beispielsweise benötigt, wenn Isobuten als Einsatzstoff zur Herstellung von Isobuten-haltigen Kunststoffen verwendet wird. Bei diesen Herstellungsverfahren sind im Allgemeinen Reinheitsanforderungen an den Ausgangsstoff gegeben, wie z.B. Mindestgehalt von Isobuten, sowie Maximalgehalt von 1-Buten und anderen Komponenten wie Butan, C3- und C5-Kohlenwasserstoffe. Eine besondere Herausforderung stellt bei der Herstellung von Isobuten-angereicherten Produkten aus einem C4-Strom, der verschiedene Kohlenwasserstoffe mit 4 Kohlenstoffatomen pro Molekül wie z.B. 1-Buten, 2-Buten, Isobutan und Isobuten umfasst, die Auftrennung von Isobuten und 1-Buten dar, da die Siedepunkte von Isobuten und 1-Buten sehr nahe beieinander liegen, wodurch eine Auftrennung mithilfe von Destillation nur durch verhältnismäßig hohem Energieaufwand möglich ist. Demgegenüber ist die Auftrennung von Isobuten und 2-Buten aufgrund der größeren Differenz der Siedepunkte dieser beiden Buten-Verbindungen einfacher. Im Stand der Technik sind deshalb Verfahren bekannt, die im Wesentlichen darauf beruhen, dass das 1-Buten im C4-Strom zu 2-Buten isomerisiert wird, um die Abtrennung von Isobuten aus dem C4-Strom zu erleichtern.

So offenbart die GB 570,692 ein Verfahren zur Abtrennung von Isobuten, bei dem im Wesentlichen ein C4-Strom, der Isobuten und 1-Buten beinhaltet, mit einem Katalysator, der die Isomerisierung von 1-Buten zu 2-Buten beschleunigt, bei einer ausreichend hohen Raumgeschwindigkeit in Berührung gebracht wird, sodass 1-Buten in 2-Buten, jedoch nicht Isobuten, umgewandelt wird und Isobuten durch Fraktionierung gewonnen wird. Um die Reinheit des Isobutens im Produktstrom des Verfahrens zu erhöhen, können die katalytische Isomerisierung in einem Reaktor und die Fraktionierung in einer Rektifikationskolonne wiederholt werden, wobei der Energieverbrauch des Herstellungsprozesses jedoch deutlich erhöht wird. Darüber hinaus ist nachteilig, dass die Reinheit des mit diesem Verfahren erzeugten Isobutens für zahlreiche Anwendungen zu gering ist, da der Massenanteil von 1-Buten nach der Isomerisierung zu hoch ist, um die notwendige Reinheit des Isobutens in der darauffolgenden Fraktionierung zu erreichen, da das 1-Buten im Isobuten-haltigen Produktstrom angereichert wird.

Die EP 0922 018 B1 zeigt ein Verfahren zur Herstellung von Isobuten aus einem C4-Strom, der Isobuten und 1-Buten enthält. Dabei wird der C4-Strom in einer Destillationskolonne behandelt, wobei der Destillationskolonne ein Teil der in der Destillationskolonne strömenden Flüssigkeit entnommen wird und einem Reaktor zur Isomerisierung von 1-Buten zu 2-Buten zugeführt wird. Im Reaktor wird mithilfe eines Katalysators die Isomerisierung von 1-Buten zu 2-Buten im Verhältnis zur Isomerisierung von Isobuten bevorzugt. Nachteilig ist jedoch, dass die Reinheit des Isobutens im Produktstrom für bestimmte Weiterverwendungen des Produktstroms zu gering ist.

Weitere Verfahren sind unter anderem aus US 6,215,036 B1, US 5,536,887 A, DE 198 01 089 A1 und EP 1 200 378 B1 bekannt.

Die Aufgabe der vorliegenden Erfindung besteht darin, zumindest einzelne Nachteile von bekannten Verfahren zur Aufreinigung von Isobuten aus einem C4-Strom zu lindern bzw. zu beheben. Die Erfindung setzt sich insbesondere zum Ziel, ein Verfahren und eine verfahrenstechnische Anlage zur effizienten Aufreinigung von Isobuten aus einem C4-Gemisch zu schaffen bzw. höchste Isobuten-Reinheitsgrade zu erzielen.

Die vorliegende Erfindung stellt ein Verfahren zur Aufreinigung von Isobuten aus einem C4-Strom, der zumindest 1-Buten, 2-Buten, Isobutan und Isobuten aufweist, zur Verfügung, bei dem zumindest die folgenden Schritte durchgeführt werden:
- Zuführen eines aus dem C4-Strom gewonnenen, mit Isobutan und Isobuten angereicherten Stoffstroms und eines Wasserstoff-Stroms in einen Isomerisierungsreaktor, wobei der Isomerisierungsreaktor einen Katalysator aufweist, der mindestens ein hydrieraktives Metall auf einem Träger, vorzugsweise einem Aluminiumoxidträger, aufweist;
- Inkontaktbringen des mit Isobutan und Isobuten angereicherten Stoffstroms und des Wasserstoff-Stroms mit dem Katalysator im Isomerisierungsreaktor, wodurch im mit Isobutan und Isobuten angereicherten Stoffstrom vorhandenes 1-Buten zu 2-Buten isomerisiert wird;
- Zuführen eines Produktstroms des Isomerisierungsreaktors, der im Verhältnis weniger 1-Buten aufweist als der mit Isobutan und Isobuten angereicherten Stoffstrom, in eine Rektifikationskolonne; und
- Bereitstellen eines mit Isobuten angereicherten Stoffstroms
   o durch Abtrennen dieses Stoffstroms
      ▪ über einen Seitenabzug der Rektifikationskolonne oder
      ▪ über einen Seitenabzug und als Sumpfprodukt der Rektifikationskolonne oder
      ▪ als Sumpfprodukt der Rektifikationskolonne wobei ferner ein mit Isobutan angereicherter Stoffstrom als Kopfprodukt der Rektifikationskolonne abgetrennt wird, oder
   o indem ein mit Isobutan angereichertes Kopfprodukt der Rektifikationskolonne, welches neben dem Isobutan Isobuten enthält, einer zweiten Rektifikationskolonne zugeführt wird, um das Isobutan vom Isobuten abzutrennen und den mit Isobuten angereicherten Stoffstrom im Sumpfprodukt der zweiten Rektifikationskolonne zu erhalten,
wobei der C4-Strom, aus dem der mit Isobutan und Isobuten angereicherte Stoffstrom gewonnen wird, einer weiteren Rektifikationskolonne, der ein weiterer Katalysator zugeordnet ist, der mindestens ein hydrieraktives Metall auf einem Träger, vorzugsweise einem Aluminiumoxidträger, aufweist, zugeführt wird, wobei der C4-Strom in der weiteren Rektifikationskolonne destillativ aufgetrennt wird und ein weiterer Wasserstoff-Strom und ein 1-Buten-haltiger Produktstrom der weiteren Rektifikationskolonne mit dem weiteren Katalysator in Kontakt gebracht werden, wodurch im genannten Produktstrom vorhandenes 1-Buten zu 2-Buten isomerisiert wird, wobei der Reaktionsproduktstroms des weiteren Katalysators in der weiteren Rektifikationskolonne destillativ aufgetrennt wird und ein mit 2-Buten angereicherter Stoffstrom als Sumpfprodukt der weiteren Rektifikationskolonne abgetrennt wird und der mit Isobutan und Isobuten angereicherte Stoffstrom als Kopfprodukt der weiteren Rektifikationskolonne abgetrennt wird und in den Isomerisierungsreaktor zugeführt wird, wobei dieser Stoffstrom im Verhältnis weniger 1-Buten und 2-Buten aufweist als der C4-Strom.

Dadurch wird die genannte Aufgabe gelöst.

Dementsprechend stellt die Erfindung auch eine verfahrenstechnische Anlage zur Aufreinigung von Isobuten aus einem C4-Strom, der zumindest 1-Buten, 2-Buten, Isobutan und Isobuten enthält, zur Verfügung. Diese Anlage weist zumindest auf:
- einen Isomerisierungsreaktor, der einen Katalysator aufweist, der mindestens ein hydrieraktives Metall auf einem Träger, vorzugsweise einem Aluminiumoxidträger, aufweist; und
- eine Rektifikationskolonne, die dem Isomerisierungsreaktor nachgeschaltet ist,
wobei die verfahrenstechnische Anlage eine weitere Rektifikationskolonne, der ein weiterer Katalysator zugeordnet ist, der mindestens ein hydrieraktives Metall auf einem Träger, vorzugsweise einem Aluminiumoxidträger, aufweist, aufweist, wobei der Isomerisierungsreaktor dem Kopf der weiteren Rektifikationskolonne nachgeschaltet ist
Auch dadurch wird die genannte Aufgabe gelöst.

Überraschenderweise kann mit dem erfindungsgemäßen Verfahren aufgereinigtes Isobuten mit einem Massenanteil des Isobutens von mindestens 95 %, bevorzugt mindestens 98 %, besonders bevorzugt mindestens 99 % hergestellt werden kann.

Ein aus dem C4-Strom gewonnener, mit Isobutan und Isobuten angereicherte Stoffstrom und ein Wasserstoff-Strom werden einem Isomerisierungsreaktor zugeführt, der bevorzugt wie in der EP 2 170 494 B1 offenbart ausgeführt ist, um das im mit Isobutan und Isobuten angereicherten Stoffstrom vorhandene 1-Buten zu 2-Buten in Gegenwart von Wasserstoff katalytisch zu isomerisieren. Für den Zweck der beschleunigten Isomerisierung weist der Isomerisierungsreaktor einen Katalysator auf, der mindestens ein hydrieraktives Metall auf einem Träger umfasst. Das 1-Buten des mit Isobutan und Isobuten angereicherten Stoffstroms wird mit Wasserstoff des Wasserstoff-Stroms in Gegenwart des Katalysators im Isomerisierungsreaktor zu 2-Buten isomerisiert, um den Anteil des 1-Butens im Produktstrom des Isomerisierungsreaktors weiter zu reduzieren. Dieser Produktstrom wird einer Rektifikationskolonne zugeführt, wobei ein mit Isobutan angereicherten Stoffstrom als Kopfprodukt destillativ abgetrennt wird. Ein mit Isobuten angereicherte Stoffstrom kann über vier Varianten von der Rektifikationskolonne erhalten werden. Bei der ersten, zweiten bzw. dritten Variante wird ein mit Isobuten angereicherter Stoffstrom über einen Seitenabzug, einen Seitenabzug und als Sumpfprodukt, bzw. als Sumpfprodukt der Rektifikationskolonne gewonnen. Bei der vierten Variante wird ein Stoffstrom, der Isobutan und Isobuten enthält, als Kopfprodukt der Rektifikationskolonne einer zweiten Rektifikationskolonne zugeführt, um das Isobutan vom Isobuten abzutrennen und einen mit Isobutan angereicherten Stoffstrom als Kopfprodukt und einen mit Isobuten angereicherten Stoffstrom als Sumpfprodukt der zweiten Rektifikationskolonne zu erhalten.

Der C4-Strom, aus dem der mit Isobutan und Isobuten angereicherte Stoffstrom gewonnen wird, wird einer weiteren Rektifikationskolonne, der ein weiterer Katalysator zugeordnet ist, der mindestens ein hydrieraktives Metall auf einem Träger, vorzugsweise einem Aluminiumoxidträger, aufweist, zugeführt. Der C4-Strom wird in der weiteren Rektifikationskolonne destillativ aufgetrennt, wobei ein 1-Buten-haltiger Produktstrom entsteht. Das 1-Buten, das im genannten Produktstrom enthalten ist, wird durch Kontakt dieses Produktstroms mit einem weiteren Wasserstoff-Strom in Gegenwart des weiteren Katalysators zu 2-Buten isomerisiert. Das hydrieraktive Metall des weiteren Katalysators beschleunigt die Reaktion von 1-Buten zu 2-Buten. Das Produkt dieser katalytischen Reaktion, der Reaktionsproduktstrom, wird in der weiteren Rektifikationskolonne destillativ aufgetrennt. Zumindest Teile der bei dieser Rektifikation (Gegenstromdestillation) entstehenden Produktströme werden erneut mit dem Wasserstoff des weiteren Wasserstoff-Stroms in Gegenwart des weiteren Katalysators in Kontakt gebracht, um das in diesen Produktströmen vorhandene 1-Buten zu 2-Buten zu isomerisieren. Der bei dieser katalytischen Reaktion entstehende Reaktionsproduktstrom wird wieder in der weiteren Rektifikationskolonne destillativ aufgetrennt. Diese zumindest einmalige Wiederholung der Verfahrensschritte Gegenstromdestillation und katalytische Reaktion gewährleistet eine geringe Konzentration von 1-Buten in den nachfolgenden Verfahrensschritten. Im Sumpf der weiteren Rektifikationskolonne wird ein Stoffstrom abgetrennt, der mit 2-Buten aus dem C4-Strom und aus der katalytischen Isomerisierung des 1-Butens angereichert ist. Der mit Isobutan und Isobuten angereicherte Stoffstrom wird als Kopfprodukt der weiteren Rektifikationskolonne abgetrennt, sodass der Kopf der weiteren Rektifikationskolonne mit dem Isomerisierungsreaktor verbunden ist. Der mit Isobutan und Isobuten angereicherte Stoffstrom weist im Verhältnis weniger 1-Buten und 2-Buten auf als der C4-Strom, der als Einspeisung der weiteren Rektifikationskolonne zugeführt wird.

Die verfahrenstechnische Anlage weist eine weitere Rektifikationskolonne auf, der ein weiterer Katalysator zugeordnet ist, der mindestens ein hydrieraktives Metall auf einem Träger, vorzugsweise einem Aluminiumoxidträger, aufweist, wobei der Isomerisierungsreaktor dem Kopf der weiteren Rektifikationskolonne nachgeschaltet ist.

Bei einer bevorzugten Ausführungsvariante ist der weitere Katalysator zumindest teilweise außerhalb der weiteren Rektifikationskolonne in einem Reaktor vorgesehen, wobei der Reaktor durch eine hinführende Verbindung und durch eine rückführende Verbindung mit der weiteren Rektifikationskolonne verbunden ist. Dabei wird ein 1-Buten-haltiger Produktstrom, der bei der destillativen Auftrennung in der weiteren Rektifikationskolonne entsteht, über einen Seitenabzug von der weiteren Rektifikationskolonne abgetrennt und dem Reaktor über die hinführende Verbindung zugeführt. Zusätzlich wird dem Reaktor der weitere Wasserstoff-Strom zur Bereitstellung des für die Isomerisierung benötigten Wasserstoffs zugeführt. Der bei der Isomerisierung am weiteren Katalysator entstehende Reaktionsproduktstrom wird über die rückführende Verbindung der weiteren Rektifikationskolonne zugeführt und dort destillativ aufgetrennt.

Bei einer weiteren bevorzugten Ausführungsvariante ist der weitere Katalysator in der weiteren Rektifikationskolonne in zumindest einer Trennstufe der weiteren Rektifikationskolonne vorgesehen. Dabei werden der C4-Strom und der weitere Wasserstoff-Strom in die weitere Rektifikationskolonne zugeführt und ein 1-Buten-haltiger Produktstrom, der bei der destillativen Auftrennung in der weiteren Rektifikationskolonne entsteht, innerhalb der weiteren Rektifikationskolonne dem weiteren Katalysator zugeführt und das 1-Buten dieses Produktstroms in Gegenwart von Wasserstoff zu 2-Buten isomerisiert. Der bei dieser katalytischen Isomerisierung innerhalb der weiteren Rektifikationskolonne entstehende Reaktionsproduktstrom wird in der weiteren Rektifikationskolonne destillativ aufgetrennt.

Um eine hohe Reinheit des gewonnenen Isobutens zu erzielen ist es günstig, wenn der Massenanteil des Isobutens im C4-Strom, der der weiteren Rektifikationskolonne zugeführt wird, mindestens 10 %, bevorzugt mindestens 15 %, insbesondere mindestens 20 %, ist.

Weiters ist für eine hohe Reinheit des gewonnenen Isobutens günstig, wenn der Massenanteil des 1-Butens im C4-Strom, der der weiteren Rektifikationskolonne zugeführt wird, maximal 40 %, bevorzugt maximal 30 %, insbesondere maximal 25 %, ist.

Weiters ist für eine hohe Reinheit des gewonnenen Isobutens günstig, wenn der Massenanteil des 2-Butens im C4-Strom, der der weiteren Rektifikationskolonne zugeführt wird, maximal 60 %, bevorzugt maximal 50 %, insbesondere maximal 40 %, ist.

Um eine Isomerisierung des 1-Butens zu 2-Buten im Verhältnis zur Isomerisierung des Isobutens besonders zu bevorzugen ist es günstig, wenn die Temperatur im Isomerisierungsreaktor zwischen 20 °C und 130 °C, bevorzugt zwischen 30 °C und 80 °C, insbesondere zwischen 40 °C und 70 °C, beträgt.

Gemäß einer bevorzugten Ausführungsform ist der Druck im Isomerisierungsreaktor zwischen 3 bar und 30 bar, bevorzugt zwischen 5 bar und 20 bar, insbesondere zwischen 7 bar und 16 bar, um eine Isomerisierung des 1-Butens zu 2-Buten im Verhältnis zur Isomerisierung des Isobutens besonders zu bevorzugen.

Gemäß einer weiteren bevorzugten Ausführungsform wird der mit Isobutan und Isobuten angereicherte Stoffstrom in einem Kondensator der weiteren Rektifikationskolonne zumindest zu einem Teil verflüssigt, um eine flüssige Phase oder eine Mischphase dieses Stoffstroms zu erhalten. Des Weiteren ist es günstig, wenn der Isomerisierungsreaktor eine Misch- und Verteilereinrichtung aufweist. Besonders bevorzugt werden über diese Misch- und Verteilereinrichtung der mit Isobutan und Isobuten angereicherte Stoffstrom der weiteren Rektifikationskolonne als flüssige Phase oder als Mischphase und der Wasserstoff-Strom als gasförmige Phase gleichmäßig mit dem Katalysator des Isomerisierungsreaktors in Kontakt gebracht, um eine möglichst hohe Vermischung des mit Isobutan und Isobuten angereicherten Stoffstroms mit dem Wasserstoff-Strom zu gewährleisten. Solche Misch- und Verteilereinrichtungen sind im Stand der Technik hinlänglich bekannt, so dass sich nähere Ausführungen dazu erübrigen können.

Zum Erzielen einer hohen Isomerisierungsrate ist es günstig, wenn beim Zuführen in den Isomerisierungsreaktor der Wasserstoff-Strom pro t/h des Isobutan und Isobuten angereicherten Stoffstroms zwischen 0,02 Nm³/h und 200 Nm³/h, bevorzugt zwischen 0,1 Nm³/h und 50 Nm³/h, insbesondere zwischen 0,5 Nm³/h und 5 Nm³/h, ist. Bei diesem Verhältnis ist gewährleistet, dass 1-Buten zu 2-Buten bei einer hohen Isomerisierungsrate isomerisiert wird und die Reaktionsrate der Nebenreaktion des Isobutens mit Wasserstoff zu Isobutan minimiert wird.

Gemäß einer bevorzugten Ausführungsform weist die Rektifikationskolonne zwischen 100 und 220 Trennstufen, bevorzugt zwischen 120 und 200 Trennstufen, insbesondere zwischen 130 und 180 Trennstufen auf. Des Weiteren ist es günstig, wenn das Rücklaufverhältnis Rücklauf zu Destillat der Rektifikationskolonne mindestens 10:1, bevorzugt mindestens 15:1, insbesondere mindestens 20:1, ist.

Um die Ausbeute von aufgereinigtem Isobuten bei gleichbleibendem C4-Stoffeinsatz zu erhöhen ist es günstig, wenn das Sumpfprodukt der Rektifikationskolonne zumindest teilweise als Recyclingstrom dem C4-Strom zugeführt wird. Für diesen Zweck ist der Sumpf der Rektifikationskolonne mit der Leitung verbunden, die für die Führung des C4-Stroms vorgesehen ist, der in die weitere Rektifikationskolonne eingespeist wird.

Gemäß einer weiteren bevorzugten Ausführungsform wird der mit Isobuten angereicherte Stoffstrom, der aus der Rektifikationskolonne gewonnen wird, einer zusätzlichen Rektifikationskolonne zugeführt, um einen weiter aufgereinigten Isobuten-Stoffstrom zu erhalten, der im Verhältnis weniger 1-Buten und 2-Buten aufweist als der mit Isobuten angereicherte Stoffstrom. Besonders bevorzugt ist der Massenanteil des Isobutens in dem weiter aufgereinigten Isobuten-Stoffstrom der zusätzlichen Rektifikationskolonne mindestens 95 %, bevorzugt mindestens 98 %, besonders bevorzugt mindestens 99 %, insbesondere mindestens 99,5 %.

Um den Einsatz des C4-Stroms möglichst gering zu halten ist es günstig, wenn das Sumpfprodukt der zusätzlichen Rektifikationskolonne zumindest teilweise als Recyclingstrom dem C4-Strom zugeführt wird, um die Ausbeute von aufgereinigtem Isobuten bei gleichbleibendem C4-Einsatz zu erhöhen. Für diesen Zweck ist der Sumpf der zusätzlichen Rektifikationskolonne mit der Leitung verbunden, die für die Führung des C4-Stroms vorgesehen ist, der in die weitere Rektifikationskolonne eingespeist wird.

Gemäß einer weiteren bevorzugten Ausführungsform wird der mit Isobuten angereicherte Stoffstrom über einen Seitenabzug von der Rektifikationskolonne abgetrennt, wobei zwischen 8 % und 25 %, bevorzugt zwischen 10 % und 20 %, insbesondere zwischen 12 % und 15 % der gesamten Trennstufen der Rektifikationskolonne unter der Trennstufe sind, in der der Seitenabzug ist. Besonders bevorzugt beträgt der Massenanteil des Isobutens in diesem mit Isobuten angereicherten Stoffstrom mindestens 95 %, bevorzugt mindestens 98 %, besonders bevorzugt mindestens 99 %, insbesondere mindestens 99,5 %.

Zur Senkung des Energiebedarfs ist es günstig, wenn die weitere Rektifikationskolonne eine höhere Temperatur als die Rektifikationskolonne aufweist und ein Wärmetauscher mit der Rektifikationskolonne und der weiteren Rektifikationskolonne zur Wärmeübertragung zwischen der Rektifikationskolonne und der weiteren Rektifikationskolonne verbunden ist, um die Rektifikationskolonne mit Wärme von der weiteren Rektifikationskolonne zu versorgen.

Gemäß einer weiteren bevorzugten Ausführungsform ist der weitere Katalysator ein mit Al2O3 geträgerter PdO-Katalysator.

Gemäß einer weiteren bevorzugten Ausführungsform ist das hydrieraktive Metall des Katalysators des Isomerisierungsreaktors aus der 8., 9. oder 10. Gruppe des Periodensystems der Elemente, insbesondere Palladium oder Platin. Besonders bevorzugt ist das hydrieraktive Metall dieses Katalysators Palladium mit einem Massenanteil zwischen 0,01 % und 5 %, bevorzugt zwischen 0,1 % und 0,7 %, insbesondere zwischen 0,2 % und 0,5 %.

Gemäß einer besonders bevorzugten Ausführungsform weist der Aluminiumoxidträger des Katalysators des Isomerisierungsreaktors kugelförmige Formkörper auf.

Gemäß einer weiteren bevorzugten Ausführungsform weist der Aluminiumoxidträger des Katalysators des Isomerisierungsreaktors Extrudate oder Tabletten auf.

Die Erfindung wird nachstehend anhand in den Zeichnungen gezeigten, nicht einschränkenden Ausführungsbeispielen weiter erläutert.
Fig. 1 zeigt ein Verfahrensfließschema einer erfindungsgemäßen verfahrenstechnischen Anlage, bei welcher Isobuten aus einem C4-Strom aufgereinigt wird.
Fig. 2 zeigt ein Verfahrensfließschema einer weiteren Ausführungsform der verfahrenstechnischen Anlage.
Fig. 3 zeigt ein Verfahrensfließschema einer dritten Ausführungsform der verfahrenstechnischen Anlage.
Fig. 4 zeigt ein Verfahrensfließschema einer vierten Ausführungsform der verfahrenstechnischen Anlage.

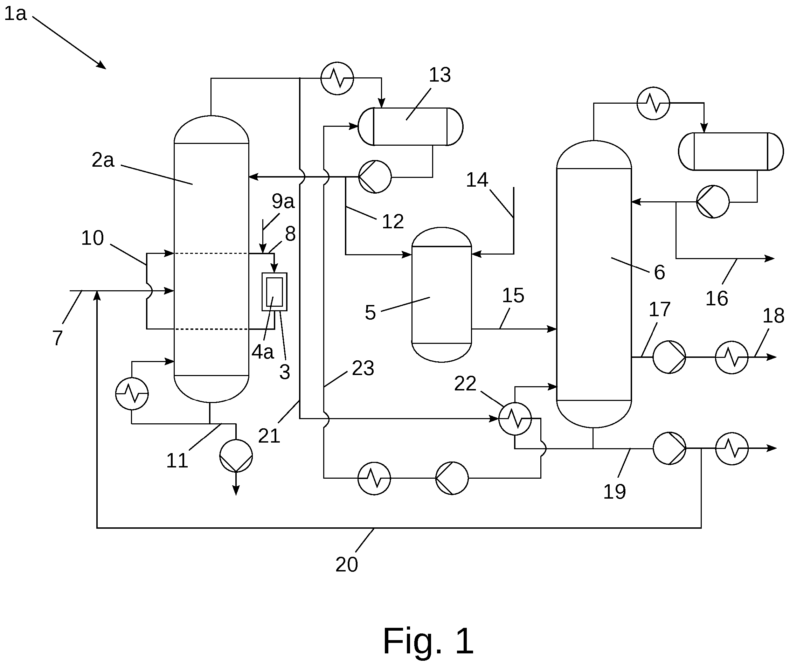

In Fig. 1 ist ein Verfahrensfließschema einer erfindungsgemäßen Anlage 1 gezeigt, die einen Isomerisierungsreaktor 5, eine Rektifikationskolonne 6, eine weitere Rektifikationskolonne 2a und einen Reaktor 3, der einen weiteren Katalysator 4a beinhaltet, aufweist. Der weitere Katalysator 4a im Reaktor 3 und die weitere Rektifikationskolonne 2a dienen der wiederholten Isomerisierung und destillativen Abtrennung von 1-Buten in einem C4-Strom, wobei sich der Reaktor 3, der den weiteren Katalysator 4a zur Isomerisierung des 1-Butens umfasst, außerhalb der weiteren Rektifikationskolonne 2a befindet. Verfahren der wiederholten Isomerisierung und destillativen Abtrennung von 1-Buten in einem C4-Strom mit einer Rektifikationskolonne und einem sich zumindest teilweise außerhalb der Rektifikationskolonne befindlichen Katalysator sind beispielsweise aus der EP 0922 018 B1 bekannt.

In der gezeigten Ausführungsform gemäß Fig. 1 wird ein C4-Strom 7, dessen Massenanteile 10 bis 40 % Isobutan, 10 bis 20 % Isobuten, 4 bis 10 % 1-Buten und 30 bis 60 % 2-Buten sein können, kontinuierlich in die weitere Rektifikationskolonne 2a eingespeist. In der weiteren Rektifikationskolonne 2a wird der C4-Strom 7 destillativ aufgetrennt und ein in der weiteren Rektifikationskolonne 2a gebildeter 1-Buten-haltiger Produktstrom 8 über zumindest einen Seitenabzug aus der weiteren Rektifikationskolonne 2a entfernt. Dieser Produktstrom 8 wird kontinuierlich einem Reaktor 3 über eine Leitung zusammen mit einem weiteren Wasserstoff-Strom 9a zugeführt, wobei sich der Reaktor 8 außerhalb der weiteren Rektifikationskolonne 2a befindet und den weiteren Katalysator 4a aufweist. Der Produktstrom 8 wird von dem weiteren Wasserstoff-Strom 9a getrennt in den Reaktor 3 zugeführt, wobei dieser Produktstrom 8 auch mit dem weiteren Wasserstoff-Strom 9a vor dem Zuführen in den Reaktor 3 gemischt werden kann. Im Reaktor 3 reagiert das 1-Buten, das im Produktstrom 8 enthalten ist, mit dem Wasserstoff des weiteren Wasserstoff-Stroms 9a in Gegenwart des weiteren Katalysators 4a, der ein mit Al2O3 geträgerter PdO-Katalysator ist, zu 2-Buten. Der dabei gebildete Reaktionsproduktstrom 10, der weniger 1-Buten und mehr 2-Buten aufweist als der Produktstrom 8, wird über eine rückführende Leitung wieder der weiteren Rektifikationskolonne 2a zugeführt. Dabei kann, wie in Fig. 1 gezeigt, die Einspeisung des rückgeführten Reaktionsproduktstroms 10 in der gleichen Trennstufe der weiteren Rektifikationskolonne 2a wie die Entnahme des Produktstroms 8 erfolgen. Das Sumpfprodukt der weiteren Rektifikationskolonne 2a ist ein mit 2-Buten angereicherter Stoffstrom 11 und das Kopfprodukt ein mit Isobutan und Isobuten angereicherter Stoffstrom 12, wobei dieser Stoffstrom 12 im Vergleich weniger 1-Buten und 2-Buten aufweist als der der weiteren Rektifikationskolonne 2a zugeführte C4-Strom 7. Die Massenanteile des mit Isobutan und Isobuten angereicherten Stoffstroms 12 können z.B. ca. 48 % Isobutan, ca. 50 % Isobuten, 600 bis 1000 ppm 1-Buten und 200 bis 400 ppm 2-Buten sein.

In der gezeigten Ausführungsform gemäß Fig. 1 wird das Kopfprodukt der weiteren Rektifikationskolonne 2a, der mit Isobutan und Isobuten angereicherte Stoffstrom 12, zumindest teilweise in einem Kondensator 13 der weiteren Rektifikationskolonne 2a verflüssigt und im kontinuierlichen Betrieb einem Isomerisierungsreaktor 5 zugeführt, der dem Kopf der weiteren Rektifikationskolonne 2a nachgeschaltet und mit der weiteren Rektifikationskolonne 2a operativ verbunden ist. In diesem Zusammenhang bedeutet operativ verbunden, dass der Isomerisierungsreaktor 5 und die weitere Rektifikationskolonne 2a im Betrieb der verfahrenstechnischen Anlage, die diese Anlagenteile aufweist, derart miteinander verbunden sind, dass ein Stoff- und/oder Energieaustausch zwischen dem Isomerisierungsreaktor 5 und der weiteren Rektifikationskolonne 2a stattfinden kann. Zusätzlich wird dem Isomerisierungsreaktor 5 ein Wasserstoff-Strom 14 zugeführt. Der mit Isobutan und Isobuten angereicherte Stoffstrom 12 wird von dem Wasserstoff-Strom 14 getrennt in den Isomerisierungsreaktor 5 zugeführt, wobei dieser Stoffstrom 12 auch mit dem Wasserstoff-Strom 14 vor dem Zuführen in den Isomerisierungsreaktor 5 gemischt werden kann. Pro t/h des Isobutan und Isobuten angereicherten Stoffstroms 12 wird dem Isomerisierungsreaktor 5 zwischen 0,5 Nm³/h und 5 Nm³/h Wasserstoff über den Wasserstoff-Strom 14 zugeführt. Durch diese Dreiphasenreaktion des mit Isobutan und Isobuten angereicherten Stoffstroms 12 als flüssige Phase, des Wasserstoff-Stroms 14 als gasförmige Phase und des Katalysators des Isomerisierungsreaktors 5 als feste Phase ist im Isomerisierungsreaktor 5 eine Misch- und Verteilereinrichtung, wie sie beispielsweise aus der EP 2 170 494 B1 bekannt ist, vorhanden, um eine möglichst hohe Durchmischung der Reaktanden zu erzielen. Die Misch- und Verteilereinrichtung weist einen Trogverteiler mit trogförmigen Kanälen und Ablaufröhrchen in den trogförmigen Kanälen für die flüssige Phase und einen Verteilerboden, der sich unterhalb des Trogverteilers befindet und vertikale Tüllen aufweist, auf. Des Weiteren weist der Isomerisierungsreaktor 5 einen Katalysator mit einem horizontal angeordneten Katalysatorfestbett mit kugelförmigen Formkörpern auf, wobei der Katalysator mindestens ein hydrieraktives Metall auf einem Aluminiumoxidträger aufweist. Das hydrieraktive Metall dieses Katalysators ist Palladium mit einem Massenanteil zwischen 0,2 % und 0,5 %. Der mit Isobutan und Isobuten angereicherte Stoffstrom 12 als flüssige Phase und der Wasserstoff-Strom 14 als gasförmige Phase werden von oben nach unten im Gleichstromverfahren mithilfe der Misch- und Verteilereinrichtung über das Katalysatorfestbett des Katalysators durch den Isomerisierungsreaktor 5 geleitet, um im mit Isobutan und Isobuten angereicherte Stoffstrom 12 vorhandenes 1-Buten mithilfe von Wasserstoff katalytisch zu 2-Buten zu isomerisieren. Bei dieser Isomerisierung im Isomerisierungsreaktor 5 beträgt die Temperatur zwischen 40 °C und 70 °C und der Druck ist zwischen 7 bar und 16 bar.

In der gezeigten Ausführungsform gemäß Fig. 1 wird der Produktstrom 15 der Isomerisierung im Isomerisierungsreaktor 5 einer Rektifikationskolonne 6 zugeführt, die dem Isomerisierungsreaktor 5 nachgeschaltet und mit diesem operativ verbunden ist. Die Rektifikationskolonne 6 weist zwischen 130 und 180 Trennstufen auf und das Rücklaufverhältnis Rücklauf zu Destillat ist 20:1. In der Rektifikationskolonne 6 wird der Produktstrom 15, dessen Massenanteile ca. 48 % Isobutan, ca. 50 % Isobuten, 100 bis 200 ppm 1-Buten und 700 bis 1200 ppm 2-Buten sind, destillativ aufgetrennt. Dabei wird ein mit Isobutan angereicherter Stoffstrom als Kopfprodukt 16 von der Rektifikationskolonne 6 abgetrennt, dessen Isobutan-Massenanteil 80 bis 95 % und Isobuten-Massenanteil 5 bis 20 % beträgt. Die Rektifikationskolonne 6 weist einen Seitenabzug 17 auf, wobei zwischen 12 % und 15 % der gesamten Trennstufen der Rektifikationskolonne 6 unter der Trennstufe sind, in der der Seitenabzug 17 ist. Über diesen Seitenabzug 17 wird ein mit Isobuten angereicherter Stoffstrom 18 abgetrennt, dessen Massenanteile 99,7 % Isobuten, 0,2 % Isobutan, 200 ppm 1-Buten und 400 ppm 2-Buten sind. Im Sumpfprodukt 19 der Rektifikationskolonne 6 beträgt der Massenanteil des Isobutens ca. 98 % und der Massenanteil des 2-Butens ca. 2 %. In der gezeigten Ausführungsform gemäß Fig. 1 wird ein Teil des Sumpfprodukts 19 als Recyclingstrom 20 dem C4-Strom 7 zugeführt. Dabei sind der Sumpf der Rektifikationskolonne 6 und die Leitung, die für die Einspeisung des C4-Stroms 7 in die weitere Rektifikationskolonne 2a vorgesehen ist, durch eine Leitung zum Führen des Recyclingstroms 20 operativ miteinander verbunden.

In der gezeigten Ausführungsform gemäß Fig. 1 wird ein Stoffstrom 21, der ein Teil des Stoffstroms ist, der über den Kopf der weiteren Rektifikationskolonne 2a abgezogen wird, dem Wärmetauscher 22, der der Verdampfer der Rektifikationskolonne 6 ist, zugeführt, um Wärme an das zu verdampfende Gemisch im Sumpf der Rektifikationskolonne 6 über den Wärmetauscher 22 abzugeben. Zur Wärmeversorgung des Verdampfers der Rektifikationskolonne 6 wird die weitere Rektifikationskolonne 2a mit einer höheren Temperatur und einem höheren Druck als die Rektifikationskolonne 6 betrieben. Eine Leitung zum Führen des Stoffstroms 21 verbindet den Kopf der weiteren Rektifikationskolonne 2a mit dem Wärmetauscher 22 der Rektifikationskolonne 6. Nach der Wärmeabgabe wird ein Stoffstrom 23, der einen geringeren Energieinhalt als der Stoffstrom 21 aufweist, vom Wärmetauscher 22 in den Kondensator 13 der weiteren Rektifikationskolonne 2a zugeführt, um den für die Wärmeübertragung dem Kopf der weiteren Rektifikationskolonne 2a entnommenen Stoffstrom dem Kopf der weiteren Rektifikationskolonne 2a wieder rückzuführen. Demgemäß ist der Wärmetauscher 22 der Rektifikationskolonne 6 und der Kondensator 13 der weiteren Rektifikationskolonne 2a durch eine Leitung zum Führen des Stoffstroms 23 verbunden.

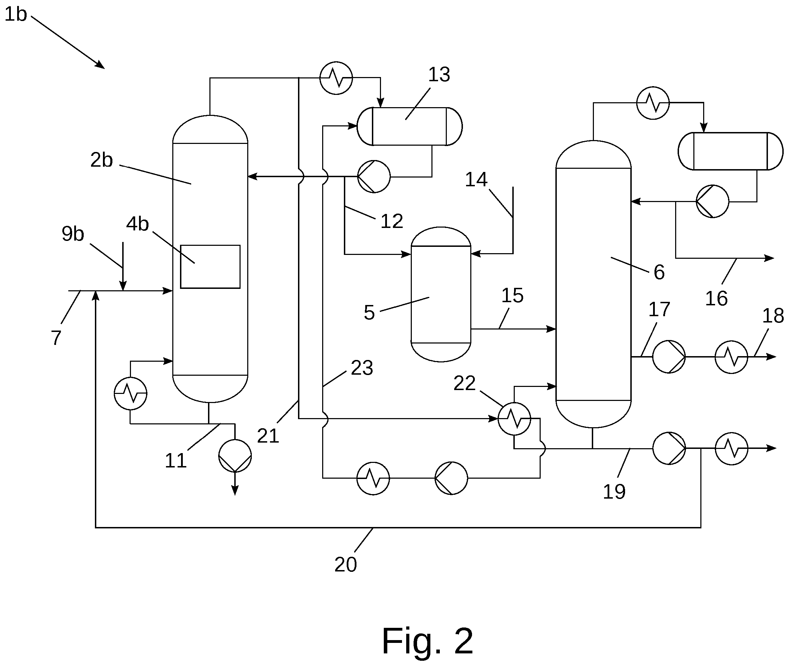

In Fig. 2 ist ein Verfahrensfließschema einer weiteren erfindungsgemäßen Anlage 1b gezeigt, die eine weitere Rektifikationskolonne 2b, einen weiteren Katalysator 4b, den Isomerisierungsreaktor 5 und die Rektifikationskolonne 6 aufweist. Der weitere Katalysator 4b und die weitere Rektifikationskolonne 2b dienen der wiederholten Isomerisierung und destillativen Abtrennung von 1-Buten in einem C4-Strom, wobei sich der weitere Katalysator 4b zur Isomerisierung des 1-Butens innerhalb der weiteren Rektifikationskolonne 2b in einer Trennstufe befindet. Verfahren der wiederholten Isomerisierung und destillativen Abtrennung von 1-Buten in einem C4-Strom mit einer Rektifikationskolonne und einem sich in der Rektifikationskolonne befindlichen Katalysator sind beispielsweise aus der EP 1 200 378 B1 bekannt.

In der gezeigten Ausführungsform gemäß Fig. 2 wird der C4-Strom 7 vor der Einspeisung in die weitere Rektifikationskolonne 2b mit einem weiteren Wasserstoff-Strom 9b gemischt und anschließend in die weitere Rektifikationskolonne 2b zugeführt. Neben der Mischung des C4-Stroms 7 mit dem weiteren Wasserstoff-Strom 9b ist auch eine getrennte Zuführung dieser beiden Ströme in die weitere Rektifikationskolonne 2b möglich. In der weiteren Rektifikationskolonne 2b wird der C4-Strom 7 destillativ aufgetrennt und der in der weiteren Rektifikationskolonne 2b gebildete 1-Buten-haltige Produktstrom 8 zusammen mit dem Wasserstoff des weiteren Wasserstoff-Stroms 9b einem weiteren Katalysator 4b zugeführt, wobei sich der weitere Katalysator 4b innerhalb der weiteren Rektifikationskolonne 2b in einer Trennstufe befindet. In Fig. 2 ist der Produktstrom 8 nicht abgebildet, da dieser einem Stoffstrom in der weiteren Rektifikationskolonne 2b entspricht, der sich in derselben Trennstufe wie der weitere Katalysator 4b befindet und mit diesem weiteren Katalysator 4b in Kontakt gebracht wird. In Gegenwart des weiteren Katalysators 4b, der ein mit Al2O3 geträgerter PdO-Katalysator ist, reagiert in der weiteren Rektifikationskolonne 2b das 1-Buten, das im Produktstrom 8 enthalten ist, mit dem Wasserstoff des weiteren Wasserstoff-Stroms 9b zu 2-Buten. Der dabei gebildete Reaktionsproduktstrom 10, der weniger 1-Buten und mehr 2-Buten aufweist als der Produktstrom 8, befindet sich ebenfalls in derselben Trennstufe wie der weitere Katalysator 4b und wird in der weiteren Rektifikationskolonne 2b destillativ aufgetrennt. Aus diesem Grund ist der Reaktionsproduktstrom 10 ebenfalls in Fig. 2 nicht abgebildet. Das Sumpfprodukt der weiteren Rektifikationskolonne 2b ist ein mit 2-Buten angereicherter Stoffstrom 11 und das Kopfprodukt ein mit Isobutan und Isobuten angereicherter Stoffstrom 12, wobei dieser Stoffstrom 12 im Vergleich weniger 1-Buten und 2-Buten aufweist als der der weiteren Rektifikationskolonne 2b zugeführte C4-Strom 7, bevor dieser C4-Strom 7 mit dem weiteren Wasserstoff-Strom 9b gemischt wird. Die weiteren Verfahrensschritte nach der weiteren Rektifikationskolonne 2b in der gezeigten Ausführungsform gemäß Fig. 2 zur Gewinnung eines mit Isobuten angereicherten Stoffstroms sind gleich den Verfahrensschritten nach der weiteren Rektifikationskolonne 2a in der Ausführungsform gemäß Fig. 1.

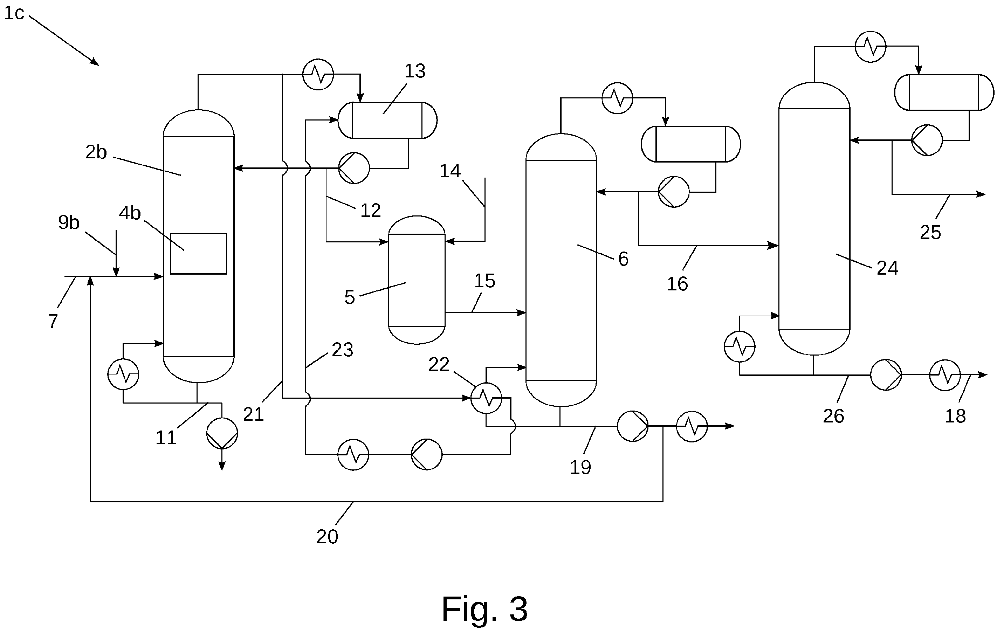

In Fig. 3 ist ein Verfahrensfließschema einer weiteren erfindungsgemäßen Anlage 1c gezeigt, die die weitere Rektifikationskolonne 2b, den weiteren Katalysator 4b, den Isomerisierungsreaktor 5 und die Rektifikationskolonne 6 gemäß der Ausführungsvariante gemäß Fig. 2 aufweist. Im Unterschied zur Ausführungsvariante gemäß Fig. 2 weist die Rektifikationskolonne 6 in der gezeigten Ausführungsform gemäß Fig. 3 keinen Seitenabzug 17 zur Abtrennung eines mit Isobuten angereicherten Stoffstroms 18 auf. Das Kopfprodukt 16 der Rektifikationskolonne 6 wird einer zweiten Rektifikationskolonne 24 zugeführt, die dem Kopf der Rektifikationskolonne 6 nachgeschaltet ist. Aus diesem Grund sind der Kopf der Rektifikationskolonne 6 und die zweite Rektifikationskolonne 24 operativ miteinander verbunden.

In der gezeigten Ausführungsform gemäß Fig. 3 wird im Vergleich zur Ausführungsvariante gemäß Fig. 2 die Rektifikationskolonne 6 mit einem höheren Druck und/oder einer höheren Temperatur betrieben, sodass ein mit Isobutan und Isobuten angereicherter Stoffstrom als Kopfprodukt 16 von der Rektifikationskolonne 6 abgetrennt wird. Dieses Kopfprodukt 16 wird der zweiten Rektifikationskolonne 24 zugeführt, um das Isobuten vom Isobutan abzutrennen und einen mit Isobutan angereicherten Stoffstrom im Kopfprodukt 25 und einen mit Isobuten angereicherten Stoffstrom 18 im Sumpfprodukt 26 der zweiten Rektifikationskolonne 24 zu erhalten. Der Massenanteil des Isobutens in diesem mit Isobuten angereicherten Stoffstrom 18 beträgt mindestens 99 %.

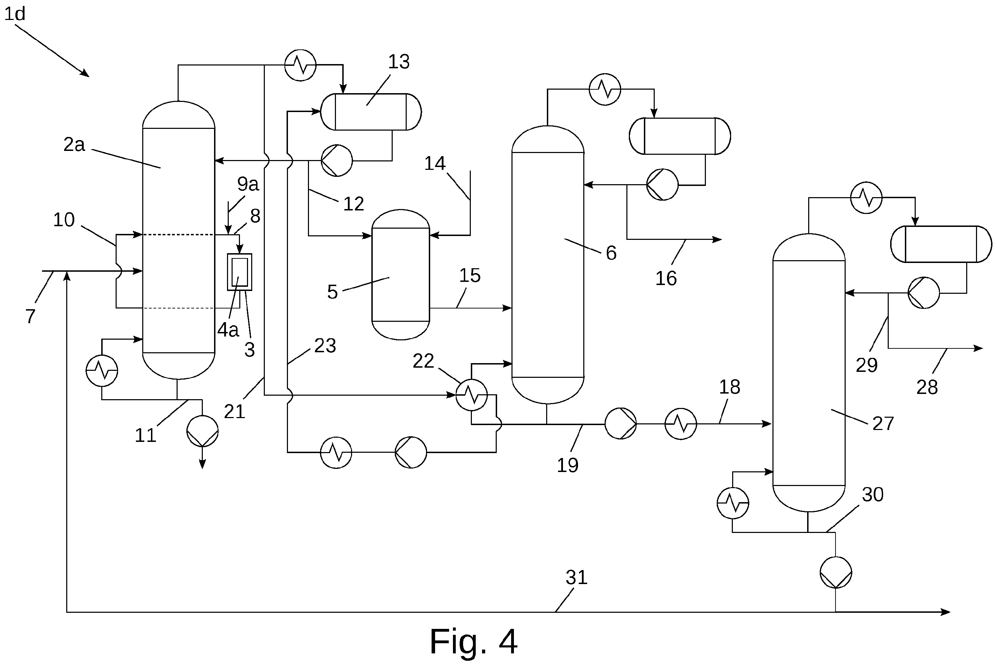

In Fig. 4 ist ein Verfahrensfließschema einer weiteren erfindungsgemäßen Anlage 1d gezeigt, die die weitere Rektifikationskolonne 2a, den Reaktor 3, der den weiteren Katalysator 4a beinhaltet, den Isomerisierungsreaktor 5 und die Rektifikationskolonne 6 gemäß der Ausführungsvariante gemäß Fig. 1 aufweist. Im Unterschied zur Ausführungsvariante gemäß Fig. 1 weist die Rektifikationskolonne 6 in der gezeigten Ausführungsform gemäß Fig. 4 keinen Seitenabzug 17 zur Abtrennung eines mit Isobuten angereicherten Stoffstroms 18 auf. Das Sumpfprodukt 19 der Rektifikationskolonne 6 wird einer zusätzlichen Rektifikationskolonne 27 zugeführt, die dem Sumpf der Rektifikationskolonne 6 nachgeschaltet ist. Aus diesem Grund sind der Sumpf der Rektifikationskolonne 6 und die zusätzliche Rektifikationskolonne 27 operativ miteinander verbunden.

In der gezeigten Ausführungsform gemäß Fig. 4 wird der als Sumpfprodukt 19 der Rektifikationskolonne 6 mit Isobuten angereicherte Stoffstrom 18 der zusätzlichen Rektifikationskolonne 27 zugeführt. In der zusätzlichen Rektifikationskolonne 27 wird das Isobuten vom 2-Buten des mit Isobuten angereicherte Stoffstroms 18 aufgetrennt und ein weiter aufgereinigter Isobuten-Stoffstrom 28 als Kopfprodukt 29 der zusätzlichen Rektifikationskolonne 27 gewonnen. Der Massenanteil des Isobutens in diesem weiter aufgereinigten Isobuten-Stoffstrom 28 beträgt mindestens 99,7 %. Das Sumpfprodukt 30 der zusätzlichen Rektifikationskolonne 27, das Isobuten und 2-Buten aufweist, wird teilweise als Recyclingstrom 31 dem C4-Strom 7 zugeführt. Dabei sind der Sumpf der zusätzlichen Rektifikationskolonne 27 und die Leitung, die für die Einspeisung des C4-Stroms 7 in die weitere Rektifikationskolonne 2a vorgesehen ist, durch eine Leitung zum Führen des Recyclingstroms 31 operativ miteinander verbunden.

## Patentansprüche

1. Verfahren zur Aufreinigung von Isobuten aus einem C4-Strom (7), der zumindest 1-Buten, 2-Buten, Isobutan und Isobuten aufweist, umfassend die folgenden Schritte:
- Zuführen eines aus dem C4-Strom (7) gewonnenen, mit Isobutan und Isobuten angereicherten Stoffstroms (12) und eines Wasserstoff-Stroms (14) in einen Isomerisierungsreaktor (5), wobei der Isomerisierungsreaktor (5) einen Katalysator aufweist, der mindestens ein hydrieraktives Metall auf einem Träger, vorzugsweise einem Aluminiumoxidträger, aufweist;
- Inkontaktbringen des mit Isobutan und Isobuten angereicherten Stoffstroms (12) und des Wasserstoff-Stroms (14) mit dem Katalysator im Isomerisierungsreaktor (5), wodurch im mit Isobutan und Isobuten angereicherten Stoffstrom (12) vorhandenes 1-Buten zu 2-Buten isomerisiert wird;
- Zuführen eines Produktstroms (15) des Isomerisierungsreaktors (5), der im Verhältnis weniger 1-Buten aufweist als der mit Isobutan und Isobuten angereicherten Stoffstrom (12), in eine Rektifikationskolonne (6); und
- Bereitstellen eines mit Isobuten angereicherten Stoffstroms (18)
o durch Abtrennen dieses Stoffstroms (18)
▪ über einen Seitenabzug (17) der Rektifikationskolonne (6) oder
▪ über einen Seitenabzug (17) und als Sumpfprodukt (19) der Rektifikationskolonne (6) oder
▪ als Sumpfprodukt (19) der Rektifikationskolonne (6)
wobei ferner ein mit Isobutan angereicherter Stoffstrom als Kopfprodukt (16) der Rektifikationskolonne (6) abgetrennt wird, oder
o indem ein mit Isobutan angereichertes Kopfprodukt (16) der Rektifikationskolonne (6), welches neben dem Isobutan Isobuten enthält, einer zweiten Rektifikationskolonne (24) zugeführt wird, um das Isobutan vom Isobuten abzutrennen und den mit Isobuten angereicherten Stoffstrom (18) im Sumpfprodukt (26) der zweiten Rektifikationskolonne (24) zu erhalten,
wobei der C4-Strom (7), aus dem der mit Isobutan und Isobuten angereicherte Stoffstrom (12) gewonnen wird, einer weiteren Rektifikationskolonne (2a, 2b), der ein weiterer Katalysator zugeordnet ist, der mindestens ein hydrieraktives Metall auf einem Träger, vorzugsweise einem Aluminiumoxidträger, aufweist, zugeführt wird, wobei der C4-Strom (7) in der weiteren Rektifikationskolonne (2a, 2b) destillativ aufgetrennt wird und ein weiterer Wasserstoff-Strom (9a, 9b) und ein 1-Buten-haltiger Produktstrom (8) der weiteren Rektifikationskolonne (2a, 2b) mit dem weiteren Katalysator (4a, 4b) in Kontakt gebracht werden, wodurch im genannten Produktstrom vorhandenes 1-Buten zu 2-Buten isomerisiert wird, wobei der Reaktionsproduktstroms (10) des weiteren Katalysators (4a, 4b) in der weiteren Rektifikationskolonne (2a, 2b) destillativ aufgetrennt wird und ein mit 2-Buten angereicherter Stoffstrom (11) als Sumpfprodukt der weiteren Rektifikationskolonne (2a, 2b) abgetrennt wird und der mit Isobutan und Isobuten angereicherte Stoffstrom (12) als Kopfprodukt der weiteren Rektifikationskolonne (2a, 2b) abgetrennt wird und in den Isomerisierungsreaktor (5) zugeführt wird, wobei dieser Stoffstrom (12) im Verhältnis weniger 1-Buten und 2-Buten aufweist als der C4-Strom (7).

2. Verfahren nach Anspruch 1, **dadurch gekennzeichnet, dass** der weitere Katalysator (4a) zumindest teilweise außerhalb der weiteren Rektifikationskolonne (2a) in einem Reaktor (3) vorgesehen ist, der Produktstrom (8) über einen Seitenabzug der weiteren Rektifikationskolonne (2a) abgetrennt wird, der Produktstrom (8) und der weitere Wasserstoff-Strom (9a) in den Reaktor (3) zugeführt werden und der Reaktionsproduktstrom (10) des weiteren Katalysators (4a) in die weitere Rektifikationskolonne (2a) zugeführt wird.

3. Verfahren nach Anspruch 1, **dadurch gekennzeichnet, dass** der weitere Katalysator (4b) in der weiteren Rektifikationskolonne (2b) in zumindest einer Trennstufe der weiteren Rektifikationskolonne (2b) vorgesehen ist, der C4-Strom (7) und der weitere Wasserstoff-Strom (9b) in die weitere Rektifikationskolonne (2b) zugeführt werden und der Reaktionsproduktstrom (10) innerhalb der weiteren Rektifikationskolonne (2b) entsteht.

4. Verfahren nach einem der Ansprüche 1 bis 3, **dadurch gekennzeichnet, dass** die Temperatur im Isomerisierungsreaktor (5) zwischen 20 °C und 100 °C, bevorzugt zwischen 30 °C und 80 °C, insbesondere zwischen 40 °C und 70 °C, beträgt.

5. Verfahren nach einem der Ansprüche 1 bis 4, **dadurch gekennzeichnet, dass** der Druck im Isomerisierungsreaktor (5) zwischen 3 bar und 30 bar, bevorzugt zwischen 5 bar und 20 bar, insbesondere zwischen 7 bar und 16 bar, beträgt.

6. Verfahren nach einem der Ansprüche 1 bis 5, **dadurch gekennzeichnet, dass** beim Zuführen in den Isomerisierungsreaktor (5) der Wasserstoff-Strom (14) pro t/h des Isobutan und Isobuten angereicherten Stoffstroms (12) zwischen 0,02 Nm³/h und 200 Nm³/h, bevorzugt zwischen 0,1 Nm³/h und 50 Nm³/h, insbesondere zwischen 0,5 Nm³/h und 5 Nm³/h, ist.

7. Verfahren nach einem der Ansprüche 1 bis 6, **dadurch gekennzeichnet, dass** das Sumpfprodukt (19) der Rektifikationskolonne (6) zumindest teilweise als Recyclingstrom (20) dem C4-Strom (7) zugeführt wird.

8. Verfahren nach einem der Ansprüche 1 bis 7, **dadurch gekennzeichnet, dass** der mit Isobuten angereicherte Stoffstrom (18) über einen Seitenabzug (17) von der Rektifikationskolonne (6) abgetrennt wird, wobei zwischen 8 % und 25 %, bevorzugt zwischen 10 % und 20 %, insbesondere zwischen 12 % und 15 % der gesamten Trennstufen der Rektifikationskolonne (6) unter der Trennstufe sind, in der der Seitenabzug (17) ist.

9. Verfahren nach einem der Ansprüche 2 bis 9, **dadurch gekennzeichnet, dass** die weitere Rektifikationskolonne (2a, 2b) eine höhere Temperatur als die Rektifikationskolonne (6) aufweist und die Rektifikationskolonne (6) mit Wärme versorgt.

10. Verfahren nach einem der Ansprüche 1 bis 9, **dadurch gekennzeichnet, dass** das hydrieraktive Metall des Katalysators des Isomerisierungsreaktors (5) aus der 8., 9. oder 10. Gruppe des Periodensystems der Elemente ist, insbesondere Palladium oder Platin ist.

11. Verfahrenstechnische Anlage (1a, 1b, 1c, 1d) zur Aufreinigung von Isobuten aus einem C4-Strom (7), der zumindest 1-Buten, 2-Buten, Isobutan und Isobuten enthält, aufweisend die folgenden Vorrichtungen:
- einen Isomerisierungsreaktor (5), der einen Katalysator aufweist, der mindestens ein hydrieraktives Metall auf einem Träger, vorzugsweise einem Aluminiumoxidträger, aufweist; und
- eine Rektifikationskolonne (6), die dem Isomerisierungsreaktor (5) nachgeschaltet ist,
**dadurch gekennzeichnet, dass** die verfahrenstechnische Anlage eine weitere Rektifikationskolonne, der ein weiterer Katalysator zugeordnet ist, der mindestens ein hydrieraktives Metall auf einem Träger, vorzugsweise einem Aluminiumoxidträger, aufweist, aufweist, wobei der Isomerisierungsreaktor dem Kopf der weiteren Rektifikationskolonne nachgeschaltet ist.

12. Verfahrenstechnische Anlage (1a, 1b, 1c, 1d) nach Anspruch 11, **dadurch gekennzeichnet, dass** sie einen Wärmetauscher (22) aufweist, der mit der Rektifikationskolonne (6) und der weiteren Rektifikationskolonne (2a, 2b) zur Wärmeübertragung zwischen der Rektifikationskolonne (6) und der weiteren Rektifikationskolonne (2a, 2b) verbunden ist.

13. Verfahrenstechnische Anlage (1a, 1b, 1c, 1d) nach einem der Ansprüche 11 oder 12, **dadurch gekennzeichnet, dass** die Rektifikationskolonne (6) einen Seitenabzug (17) zum Abtrennen eines Isobuten-haltigen Stoffstroms aufweist.

14. Verfahrenstechnische Anlage (1a, 1b, 1c, 1d) nach einem der Ansprüche 11 bis 13, **dadurch gekennzeichnet, dass** der Sumpf der Rektifikationskolonne (6) mit der für die Führung des C4-Stroms (7) vorgesehenen Leitung zum zumindest teilweise Rückführen des Sumpfprodukts (19) der Rektifikationskolonne (6) verbunden ist.

## Claims

1. A process for the purification of isobutene from a C4 stream (7) which comprises at least 1-butene, 2-butene, isobutane and isobutene, comprising the following steps:
- supplying a stream of material which is concentrated in isobutane and isobutene (12) obtained from the C4 stream (7) and a stream of hydrogen (14) to an isomerization reactor (5), wherein the isomerization reactor (5) comprises a catalyst which comprises at least one metal which is active in hydrogenation on a support, preferably an aluminium oxide support;
- bringing the stream of material which is concentrated in isobutane and isobutene (12) and the stream of hydrogen (14) into contact with the catalyst in the isomerization reactor (5), whereupon 1-butene present in the stream of material which is concentrated in isobutane and isobutene (12) is isomerized to 2-butene;
- supplying a product stream (15) which comprises less 1-butene in relation to the stream of material which is enriched with isobutane and isobutene (12) from the isomerization reactor (5) to a rectification column (6); and
- providing a stream of material which is concentrated in isobutene (18):
∘ by separating this stream of material (18):
▪ via a side stream (17) from the rectification column (6), or
▪ via a side stream (17) and as the bottom product (19) from the rectification column (6), or
▪ as a bottom product (19) from the rectification column (6),
wherein furthermore, a stream of material which is concentrated in isobutane is separated from the rectification column (6) as the overhead product (16), or
∘ in which an overhead product (16) from the rectification column (6) that is concentrated in isobutane, which contains isobutene in addition to the isobutane, is supplied to a second rectification column (24) in order to separate the isobutane from the isobutene and to obtain the stream of material which is concentrated in isobutene (18) in the bottom product (26) from the second rectification column (24),
wherein the C4 stream (7) from which the stream of material which is concentrated in isobutane and isobutene (12) is obtained is supplied to a further rectification column (2a, 2b) which is associated with a further catalyst which comprises at least one metal which is active in hydrogenation on a support, preferably on an aluminium oxide support, wherein the C4 stream (7) is separated by distillation in the further rectification column (2a, 2b) and a further hydrogen stream (9a, 9b) and a product stream which contains 1-butene (8) from the further rectification column (2a, 2b) are brought into contact with the further catalyst (4a, 4b), whereupon 1-butene present in said product stream is isomerized to 2-butene, wherein the reaction product stream (10) from the further catalyst (4a, 4b) is separated by distillation in the further rectification column (2a, 2b) and a stream of material which is concentrated in 2-butene (11) is separated from the further rectification column (2a, 2b) as the bottom product and the stream of material (12) which is concentrated in isobutane and isobutene is separated from the further rectification column (2a, 2b) as the overhead product and supplied to the isomerization reactor (5), wherein this stream of material (12) contains less 1-butene and 2-butene compared with the C4 stream (7).

2. The process as claimed in claim 1, **characterized in that** at least a portion of the further catalyst (4a) is located outside the further rectification column (2a) in a reactor (3), the product stream (8) is separated from the further rectification column (2a) via a side stream, the product stream (8) and the further stream of hydrogen (9a) are supplied to the reactor (3) and the reaction product stream (10) from the further catalyst (4a) is supplied to the further rectification column (2a).

3. The process as claimed in claim 1, **characterized in that** the further catalyst (4b) in the further rectification column (2b) is provided in at least one separation stage of the further rectification column (2b), the C4 stream (7) and the further stream of hydrogen (9b) are supplied to the further rectification column (2b) and the reaction product stream (10) is produced inside the further rectification column (2b).

4. The process as claimed in one of claims 1 to 3, **characterized in that** the temperature in the isomerization reactor (5) is between 20°C and 100°C, preferably between 30°C and 80°C, in particular between 40°C and 70°C.

5. The process as claimed in one of claims 1 to 4, **characterized in that** the pressure in the isomerization reactor (5) is between 3 bar and 30 bar, preferably between 5 bar and 20 bar, in particular between 7 bar and 16 bar.

6. The process as claimed in one of claims 1 to 5, **characterized in that** when supplied to the isomerization reactor (5), the flow rate of hydrogen (14) per t/h of the stream of material which is concentrated in isobutane and isobutene (12) is between 0.02 Nm³/h and 200 Nm³/h, preferably between 0.1 Nm³/h and 50 Nm³/h, in particular between 0.5 Nm³/h and 5 Nm³/h.

7. The process as claimed in one of claims 1 to 6, **characterized in that** at least a portion of the bottom product (19) from the rectification column (6) is supplied to the C4 stream (7) as the recycle stream (20).

8. The process as claimed in one of claims 1 to 7, **characterized in that** the stream of material which is concentrated in isobutene (18) is separated from the rectification column (6) via a side stream (17), wherein between 8% and 25%, preferably between 10% and 20%, in particular between 12% and 15% of the total separation stages of the rectification column (6) are below the separation stage in which the side stream (17) is located.

9. The process as claimed in one of claims 2 to 9, **characterized in that** the further rectification column (2a, 2b) is at a higher temperature than the rectification column (6) and the rectification column (6) is supplied with heat.

10. The process as claimed in one of claims 1 to 9, **characterized in that** the metal which is active in hydrogenation of the catalyst of the isomerization reactor (5) is from group 8, 9 or 10 of the periodic table of the elements, in particular palladium or platinum.

11. A processing facility (1a, 1b, 1c, 1d) for the purification of isobutene from a C4 stream (7) which contains at least 1-butene, 2-butene, isobutane and isobutene, comprising the following equipment:
- an isomerization reactor (5) which comprises a catalyst which comprises at least one metal which is active in hydrogenation on a support, preferably an aluminium oxide support; anda rectification column (6) which is connected downstream of the isomerization reactor (5),
**characterized in that** the processing facility comprises a further rectification column, which is associated with a catalyst which comprises at least one metal which is active in hydrogenation on a support, preferably on an aluminium oxide support, wherein the isomerization reactor is downstream of the head of the further rectification column.

12. The processing facility (1a, 1b, 1c, 1d) as claimed in claim 11, **characterized in that** it comprises a heat exchanger (22) which is connected to the rectification column (6) and the further rectification column (2a, 2b) for heat transfer between the rectification column (6) and the further rectification column (2a, 2b).

13. The processing facility (1a, 1b, 1c, 1d) as claimed in claim 11 or 12, **characterized in that** the rectification column (6) comprises a side stream (17) for separating a stream of material containing isobutene.

14. The processing facility (1a, 1b, 1c, 1d) as claimed in one of claims 11 to 13, **characterized in that** the bottom of the rectification column (6) is connected to the line provided to convey the C4 stream (7) for at least partial recycling of the bottom product (19) from the rectification column (6).

## Revendications

1. Procédé de purification d'isobutène à partir d'un flux C4 (7) contenant au moins du 1-butène, du 2-butène, de l'isobutane et de l'isobutène, comprenant les étapes suivantes :
- introduction d'un flux de matière (12) enrichi en isobutane et isobutène obtenu à partir du flux C4 (7) et d'un flux d'hydrogène (14) dans un réacteur d'isomérisation (5), le réacteur d'isomérisation (5) comprenant un catalyseur qui comporte au moins un métal hydrogénant actif sur un support, de préférence un support d'oxyde d'aluminium ;
- mise en contact du flux de matière enrichi en isobutane et isobutène (12) et du flux d'hydrogène (14) avec le catalyseur dans le réacteur d'isomérisation (5), ce qui isomère le 1-butène présent dans le flux de matière enrichi en isobutane et isobutène (12) en 2-butène ;
- introduction d'un flux de produit (15) du réacteur d'isomérisation (5), qui contient proportionnellement moins de 1-butène que le flux de matière enrichi en isobutane et isobutène (12), dans une colonne de rectification (6) ; et
- fourniture d'un flux de matière enrichi en isobutène (18)
o en séparant ce flux de matière (18)
■ via une extraction latérale (17) de la colonne de rectification (6) ou
■ via une extraction latérale (17) et comme produit de fond (19) de la colonne de rectification (6) ou
■ comme produit de fond (19) de la colonne de rectification (6)
où en outre un flux de matière enrichi en isobutane est séparé comme produit de tête (16) de la colonne de rectification (6), ou
o en introduisant un produit de tête (16) enrichi en isobutane de la colonne de rectification (6), qui contient, outre l'isobutane, de l'isobutène, dans une deuxième colonne de rectification (24) pour séparer l'isobutane de l'isobutène et obtenir le flux de matière enrichi en isobutène (18) dans le produit de fond (26) de la deuxième colonne de rectification (24),
où le flux C4 (7), à partir duquel le flux de matière enrichi en isobutane et isobutène (12) est obtenu, est introduit dans une autre colonne de rectification (2a, 2b), à laquelle est associé un autre catalyseur qui comporte au moins un métal hydrogénant actif sur un support, de préférence un support d'oxyde d'aluminium, où le flux C4 (7) est séparé par distillation dans l'autre colonne de rectification (2a, 2b) et un autre flux d'hydrogène (9a, 9b) et un flux de produit (8) contenant du 1-butène de l'autre colonne de rectification (2a, 2b) sont mis en contact avec l'autre catalyseur (4a, 4b), ce qui isomère le 1-butène présent dans ledit flux de produit en 2-butène, où le flux de produit de réaction (10) de l'autre catalyseur (4a, 4b) est séparé par distillation dans l'autre colonne de rectification (2a, 2b) et un flux de matière enrichi en 2-butène (11) est séparé comme produit de fond de l'autre colonne de rectification (2a, 2b) et le flux de matière enrichi en isobutane et isobutène (12) est séparé comme produit de tête de l'autre colonne de rectification (2a, 2b) et est introduit dans le réacteur d'isomérisation (5), ce flux de matière (12) contenant proportionnellement moins de 1-butène et de 2-butène que le flux C4 (7).

2. Procédé selon la revendication 1, **caractérisé en ce que** l'autre catalyseur (4a) est prévu au moins partiellement à l'extérieur de l'autre colonne de rectification (2a) dans un réacteur (3), le flux de produit (8) est séparé via une extraction latérale de l'autre colonne de rectification (2a), le flux de produit (8) et l'autre flux d'hydrogène (9a) sont introduits dans le réacteur (3) et le flux de produit de réaction (10) de l'autre catalyseur (4a) est introduit dans l'autre colonne de rectification (2a).

3. Procédé selon la revendication 1, **caractérisé en ce que** l'autre catalyseur (4b) est prévu dans l'autre colonne de rectification (2b) dans au moins un étage de séparation de l'autre colonne de rectification (2b), le flux C4 (7) et l'autre flux d'hydrogène (9b) sont introduits dans l'autre colonne de rectification (2b) et le flux de produit de réaction (10) se forme à l'intérieur de l'autre colonne de rectification (2b).

4. Procédé selon l'une des revendications 1 à 3, **caractérisé en ce que** la température dans le réacteur d'isomérisation (5) est comprise entre 20 °C et 100 °C, de préférence entre 30 °C et 80 °C, en particulier entre 40 °C et 70 °C.

5. Procédé selon l'une des revendications 1 à 4, **caractérisé en ce que** la pression dans le réacteur d'isomérisation (5) est comprise entre 3 bar et 30 bar, de préférence entre 5 bar et 20 bar, en particulier entre 7 bar et 16 bar.

6. Procédé selon l'une des revendications 1 à 5, **caractérisé en ce que** lors de l'introduction dans le réacteur d'isomérisation (5), le flux d'hydrogène (14) par t/h du flux de matière enrichi en isobutane et isobutène (12) est compris entre 0,02 Nm³/h et 200 Nm³/h, de préférence entre 0,1 Nm³/h et 50 Nm³/h, en particulier entre 0,5 Nm³/h et 5 Nm³/h.

7. Procédé selon l'une des revendications 1 à 6, **caractérisé en ce que** le produit de fond (19) de la colonne de rectification (6) est introduit au moins partiellement comme flux de recyclage (20) dans le flux C4 (7).

8. Procédé selon l'une des revendications 1 à 7, **caractérisé en ce que** le flux de matière enrichi en isobutène (18) est séparé via une extraction latérale (17) de la colonne de rectification (6), entre 8 % et 25 %, de préférence entre 10 % et 20 %, en particulier entre 12 % et 15 % de tous les étages de séparation de la colonne de rectification (6) étant situés sous l'étage de séparation où se trouve l'extraction latérale (17).

9. Procédé selon l'une des revendications 2 à 9, **caractérisé en ce que** l'autre colonne de rectification (2a, 2b) a une température plus élevée que la colonne de rectification (6) et alimente la colonne de rectification (6) en chaleur.

10. Procédé selon l'une des revendications 1 à 9, **caractérisé en ce que** le métal hydrogénant actif du catalyseur du réacteur d'isomérisation (5) est issu du 8ème, 9ème ou 10ème groupe du tableau périodique des éléments chimiques, en particulier du palladium ou du platine.

11. Installation de procédé (1a, 1b, 1c, 1d) pour la purification d'isobutène à partir d'un flux C4 (7) contenant au moins du 1-butène, du 2-butène, de l'isobutane et de l'isobutène, comprenant les dispositifs suivants :
- un réacteur d'isomérisation (5) qui comprend un catalyseur comportant au moins un métal hydrogénant actif sur un support, de préférence un support d'oxyde d'aluminium ; et
- une colonne de rectification (6) qui est disposée en aval du réacteur d'isomérisation (5),
**caractérisée en ce que** l'installation de procédé comprend une autre colonne de rectification, à laquelle est associé un autre catalyseur qui comporte au moins un métal hydrogénant actif sur un support, de préférence un support d'oxyde d'aluminium, où le réacteur d'isomérisation est disposé en aval de la tête de l'autre colonne de rectification.

12. Installation de procédé (1a, 1b, 1c, 1d) selon la revendication 11, **caractérisée en ce qu'**elle comprend un échangeur de chaleur (22) qui est relié à la colonne de rectification (6) et à l'autre colonne de rectification (2a, 2b) pour le transfert de chaleur entre la colonne de rectification (6) et l'autre colonne de rectification (2a, 2b).

13. Installation de procédé (1a, 1b, 1c, 1d) selon l'une des revendications 11 ou 12, **caractérisée en ce que** la colonne de rectification (6) comprend une extraction latérale (17) pour séparer un flux de matière contenant de l'isobutène.

14. Installation de procédé (1a, 1b, 1c, 1d) selon l'une des revendications 11 à 13, **caractérisée en ce que** le fond de la colonne de rectification (6) est relié à la conduite prévue pour l'acheminement du flux C4 (7) afin de recycler au moins partiellement le produit de fond (19) de la colonne de rectification (6).
